# EUROPEAN PATENT APPLICATION

(11) **EP 3 685 779 A1**
(43) Date of publication of application: **29.07.2020**
(21) Application number: 19153548.3
(22) Date of filing: 24.01.2019
(51) Int. Cl.: A61B 18/04, A61B 18/00, H05H 1/24

(54) **DEVICE FOR COLD PLASMA TREATMENT, COLD PLASMA ENDOSCOPIC SYSTEM, AND METHOD FOR GENERATING AND TRANSPORTING A COLD PLASMA**

(71) Applicant: Université Libre de Bruxelles, 1050 Bruxelles (BE)
(72) Inventor: BASTIN, Orianne, 1380 Lasne (BE); THULLIEZ, Max, 1030 Brussels (BE); DELCHAMBRE, Alain, 1170 Brussels (BE); NONCLERCQ, Antoine, 1170 Brussels (BE); DEVIERE, Jacques, 1404 Bornival (BE); BLERO, Daniel, 1310 La Hulpe (BE); HADEFI, Alia, 1090 Jette (BE); RENIERS, François, 1170 Brussels (BE); MERCHE, Delphine, 1050 Ixelles (BE); OZKAN, Alp, 1030 Schaerbeek (BE); VANDENCASTEELE, Nicolas, 13100 Saint Marc Jaumegarde (FR)
(74) Representative: Pronovem

(57) **Abstract**

Device (100) for cold plasma endoscopy comprising:
- a cold plasma generating system (10) comprising:
∘ a gas source (11),
∘ an electrical source (12),
∘ a cold plasma chamber (13) comprising:
▪ a dielectric chamber (14) fluidly connected to said gas source (11),
▪ a first electrode (15) surrounding at least partially said dielectric chamber (14) and electrically connected to said electrical energy source (12);

- a first flexible tube (20) having a first lumen (25) fluidly connected to said dielectric chamber (14);
said device (100) further comprising:
- electrically conductive means (27) positioned at least partially inside said first lumen (25).

## Description

### Technical area

According to a first aspect, the invention relates to a device for cold plasma treatment.

According to a second aspect, the invention relates to a cold plasma endoscopic system.

According to a third aspect, the invention relates to a method for generating and transporting a cold plasma.

According to a fourth aspect, the invention relates to confinement and dispensing means for a cold plasma endoscopic system for cold plasma treatment.

### Prior art

WO 2009/050240 A1 describes a plasma generation system for generating plasmas balls allowing transport over relatively long distances. These plasma balls are travelling in dielectric guide at the end of which there is an apparent plasma plume like zone, which shape and intensity depend on the discharge repetition rate. Such plasma balls are suitable for localized plasma treatment with a area to be treated being restricted.

There exists a need for providing a device for generating a continuous plasma plume transportable over long distances for the treatment of large surfaces and/or volumes.

### Summary of the invention

According to a first aspect, one of the objects of the present invention is to provide a device for plasma generation and transport with a high efficiency over a long distance.

To this end, the inventors propose a device for cold plasma treatment comprising:
- a cold plasma generating system comprising:
   ∘ a gas source,
   ∘ an electrical source,
   ∘ a cold plasma chamber comprising:
      ▪ a dielectric chamber fluidly connected to said gas source,
      ▪ a first electrode surrounding at least partially said dielectric chamber and electrically connected to said electrical source;
- a first tube comprising a first lumen fluidly connected to said dielectric chamber;
said device further comprising electrically conductive means positioned at least partially inside said first lumen. Preferably, said first tube is a first flexible tube.

One of the advantages of using a conductive wire placed at least partially in said first flexible tube and preferably placed partially in said chamber is that transport of a plasma over long distances in a flexible tube is greatly improved. Advantages of using said conductive wire are as follows:
- said wire allows transporting cold plasma over long distances;
- because said wire allows cold plasma transport over long distances, said device can be used for cold plasma treatment and more particularly for cold plasma treatment with a cold plasma endoscopic system, of a digestive system, preferably a human digestive system;
- thanks to this cold plasma generation and efficient transport of cold plasma, the device of the invention allows the use of cold plasma for new treatments;
- thanks to the cold plasma transporting means comprising a wire, cold plasma treatment can be realized without in-situ cold plasma generation, thus requiring no high-voltage in-situ nor close to the endoscopic system user;
- for a definite amount of plasma delivered, said wire allows relatively low power for cold plasma generation because the generated cold plasma is efficiently transported;
- said device for cold plasma treatment allows to have a continuous cold plasma plume at the end of the first tube;
- it is possible to design cold plasma devices that are more compact and easier to handle, because the cold plasma generation can be taken away from the cold plasma target;
- the use of a conductive wire placed partially in said chamber and at least partially in said flexible tubing allows a relatively low power cold plasma generation for a long distance transportation of the cold plasma plume.

Another advantage of the device for cold plasma treatment of the invention is to provide a good control of the cold plasma properties because of reduced losses during the cold plasma transportation through the flexible tubing. Another advantage is to be able to have a cold plasma plume at the exit of the flexible tubing, the cold plasma plume having a more stable shape and being more easily controlled. A better control of the cold plasma plume is obtained by the position of the conductive means regarding the distal end of the flexible tubing; plume length, plume stability can be better controlled. A good control of the cold plasma properties is for example a good control of the number of generated ionized species in the cold plasma plume.

Preferably, the electrical source or electrical energy source allows to apply a voltage (sinusoidal, triangular, squared or pulsed) to the electrode in order to trigger and maintain the cold plasma. Electrical source or electrical energy source means power controlled source, voltage controlled source or current controlled source. More preferably it is a high voltage source. For example it is a sinusoidal, triangular, squared, pulsed voltage source.

Preferably, said cold plasma generating chamber comprises a grounded second electrode and said electrical energy source being electrically connected to the ground. For example, for a voltage source having a neutral and a phase, the neutral of a voltage source is grounded.

Preferably, said first tube is a first flexible tube. Preferably, said first flexible tube is a polymer, a PolyTetraFluoroEthylene (PTFE) or a PolyEthylene (PE) tube having an outer diameter comprised between 1 mm and 10 mm, more preferably between 2 mm and 5 mm and for example of 3 mm. Preferably said first flexible tube has an inner diameter comprised between 0.5 mm and 8 mm, more preferably between 1 mm and 5 mm and for example of 2 mm.

Cold plasma plume means the plasma which is generated at the exit of the transporting means, or of the first tube, preferably of the first flexible tube.

Cold plasma transport over a long distance means a distance exceeding 50 cm, preferably a distance exceeding 1 m and even more preferably a distance exceeding 2 m.

The cold plasma generating device of the invention is particularly advantageous because it allows cold plasma generation and transport over a long distance. The generated cold plasma can be transported over a long distance from the cold plasma generation through a flexible tubing. Therefore the cold plasma generating device of the invention allows its use for endoscopy by using a catheter for transporting the cold plasma, the catheter being positioned inside a working channel of an endoscope. The main advantage of the present invention comes from the cold plasma generation outside a body to be penetrated by the endoscope (according to the second aspect) or by the flexible tube of the device for cold plasma treatment (according to the first aspect) through an endoscope (according to the second aspect). State of the art document US 9 192 040 B2 describes a cold plasma generator located at the end of a flexible tubing which necessitates transport of a high voltage along said flexible tubing. For applications in a living body, transport of high voltages for cold plasma generation within a living body are dangerous. Also transport of high voltages along a flexible tube requires electrical isolation means that may be relatively thick for endoscopy purposes. Also, in situ cold plasma generation would be unsuitable for endoscopic purposes because it requires a dielectric barrier discharge (DBD) which takes up space. Cold plasma treatment is much more interesting than hot plasma for certain applications, *e.g*. treatment of large portions of tissue or mucosa.

Preferably, the dielectric chamber is made in quartz. Preferably the potential applied to the first electrode (or plurality of first electrode portions) is an excitation signal having a sinusoidal potential varying in time with a frequency in the kHz range, for example comprised between 1 kHz and 1 MHz. Preferably the first electrode is in contact with the dielectric chamber.

In another embodiment, the excitation signal is pulsed with a potential applied to the first electrode varying with pulses from no potential to an ionising potential. For example the pulse width for a pulsed excitation is in the ns range, i.e. comprised between 1 ns to 1 µs, more preferably between 1 ns to 100 ns. Preferably, the pulsed excitation signal is pulsed unipolar or bipolar. Unipolar or monopolar means that all pulses are pulsed with a same polarity. Bipolar means that one pulse over two is pulsed with a different polarity. As a result, the gas originating from the gas source is ionised and forms a cold plasma in the dielectric chamber. The cold plasma then flows through the transporting means as it is pushed away by the gas entering the dielectric chamber. Preferably, the gas source is connected to the dielectric chamber opposite to the transporting means connection with the dielectric chamber such that a direct flow of gas occurs in the dielectric chamber. Preferably, the period of pulse for the above mentioned pulses is comprised between 1 kHz and 100 kHz.

Plasma can be classified in two categories according to their thermodynamical equilibrium and related temperature.
- Thermal plasma or "Local Thermodynamic Equilibrium" plasma, referred to hereafter as "hot plasma" contains species which all present a similar temperature. This plasma thus reach very high temperature of several thousand degrees or more.
- Non thermal plasma or non-LTE plasmas, often named and referred to in the present document as "cold plasma", presents only highly energetic electrons. These electrons exhibit a temperature significantly higher than the ions and neutrals, resulting in a plasma at much lower temperature (from room temperature to a few hundred degrees). This is made possible by the high mass difference between electrons and ions, lighter electrons being accelerated more easily than their counterparts and losing few energy during collisions with the heavy ions. This explains the high thermodynamic disequilibrium and the low temperature. As opposed to the thermal plasma, cold plasma contains much more neutral species than free charge carriers.

The advantage of the cold plasma is that its effect on surfaces is related to its reactive species, electric field and emissions (e.g. UV light) since the plasma itself stays at room temperature as opposed to hot plasmas for which the main effect used in medicine is related to its high temperature allowing to burn tissues. Another advantage of cold plasma is its relatively low (with respect to hot plasma) electron density, resulting in a relatively low current administration. Finally, cold plasma also allows for more widespread and homogeneous plasma (in opposition with arc discharges).

Preferably, said electrically conductive means are placed partially in said dielectric chamber and at least partially in said first lumen.

Preferably, said first electrode comprises at least two, more preferably three or more, electrode portions being separated longitudinally around said dielectric chamber and being connected to said electrical source. Electrode portions are portions of said first electrode. Said first electrode portions being connected to the electrical source with the same electric voltage.

Preferably, said electrically conductive means are electrically insulated from said first electrode.

Thanks to the electrically conductive means, the generated cold plasma is transported to long distances without the use of a high voltage wire or cable along the plasma transporting means.

Preferably, the electrically conductive means has a floating potential. In another embodiment, the potential of the electrically conductive means is fixed. A floating potential means that it is isolated from the electrical source. Floating potential means that the potential can vary depending on the surrounding plasma around. Said electrical source being preferably a voltage source.

Preferably, said electrically conductive means extend essentially all along said first lumen.

Preferably, the first flexible tubing has a first end fluidly connected to said dielectric chamber and a second end from which the transported cold plasma exits the first tube, more preferably the first flexible tube. For example, said generated and transported cold plasma forms a plume from said second end of the first tube. The electrically conductive means extending essentially all along the first tube means that it extends along at least 90 % of the length of the first tube, more preferably along at least 95 % of the length of the first tube, more preferably, the first tube is a first flexible tube.

Preferably, said electrically conductive means are an electrically conductive wire.

Preferably, said electrically conductive wire is metallic, more preferably said conductive wire is a copper wire. Said metallic or copper wire preferably has a diameter comprised between 0.05 mm and 1 mm, more preferably has a diameter comprised between 0.1 mm and 0.5 mm, for example has a diameter of 0.2 mm. In a preferred embodiment said conductive wire is deposited on the inner surface of the first tube, more preferably on the inner surface of a first flexible tube. In another preferred embodiment, said conductive wire is a layer of a conductive ink deposited on the inner surface of the first tube, more preferably of a first flexible tube.

The inventors have estimated that for the same device for cold plasma treatment, with a same first tube (first flexible tube), for creating a cold plasma plume with the same light intensity and *in fine* delivering a same quantity of ionized species at the distal end per unit of time, an electrical source with a power consumption of about 150 W is required when no electrically conductive wire is inserted in the first lumen instead of a power consumption of about 50 W when an electrically conductive wire is present in said first lumen. Therefore the invention compared to US 9 192 040 B2 allows to transport cold plasma over a long distance after its generation rather than generating a cold plasma all along the tube up its end. The object of the present invention allows to provide a better ratio of ionised species or reactive species for a same power consumption.

The use of an electrically conductive wire is particularly interesting for applications for which a limited time frame is given for applying a cold plasma to a hollow surface. For example, the device for cold plasma treatment is particularly well suited for the treatment of the duodenal tract in order to smoothly resurface its mucosa through natural ways. Thanks to the high yield of transported cold plasma at the distal end, the resurfacing of the duodenal tract would be possible in its entirety within a relatively short period of time and with a relatively low power. Similar features are applicable for the oesophagus when a mucosa, dysplastic or not, has to be ablated, or for any other surface of the digestive, urinary or pulmonologic tracts where an ablation of the mucosal surface may be of clinical interest.

Preferably, said electrically conductive wire is electrically insulated from said electrical energy source (voltage source) and more preferably from said first electrode.

Preferably, said electrically conductive wire is mechanically floating within said first lumen or said electrically conductive wire is mechanically coupled to an inner surface of said first flexible tube. In another embodiment, said conductive means is floating or suspended within a conductive means lumen being separated from the first lumen but being adjacent to it.

Mechanically floating means that the conductive wire is essentially not mechanically coupled to said inner surface of said first flexible tube delimiting said first lumen. Floating means suspended within said first lumen. Floating means that the electrically conductive wire can move freely within said first lumen.

Preferably, said electrically conductive wire is not electrically insulated within the first lumen. Said electrically conductive wire is not electrically insulated from the gas and/or plasma inside said first lumen. Said electrically conductive wire is not insulated from said first flexible tube walls, for example first flexible tube walls being in PTFE. Said electrically conductive wire is conductive within said first lumen such that it can electrically interact with a gas or a plasma present in said first lumen.

Preferably, said dielectric chamber has a rounded cross section and in that said first electrode surrounding said rounded cross section all around. Preferably, said first electrode is made of a single portion, or of multiple portions, for example electrically connected rings around said dielectric chamber. In another embodiment, said first electrode being of any shape surrounding at least partially said dielectric chamber.

According to a second aspect, the invention relates to a cold plasma endoscopic system comprising a device according to the first aspect of the invention.

The advantage of the cold plasma endoscopic system of the invention is to provide a cold plasma endoscopic system for treatment of large surfaces through the use of appropriate radially dispensing means and/or longitudinal confinement means.

Preferably said catheter is a flexible tube. Said catheter is single-lumen or multi-lumen. Preferably said catheter is made in a fluorinated polymer, for example PTFE.

The advantages described for the device for cold plasma treatment according to the first aspect of the invention also apply to the cold plasma endoscopy system according to the second aspect, *mutatis mutandis.* The different variants for the device for cold plasma treatment according to the first aspect of the invention as described in the preceding text also apply to the cold plasma endoscopy system according to the second aspect of the invention, *mutatis mutandis.*

Preferably, said cold plasma endoscopic system comprising:
- an endoscope, said endoscope having a distal end, said endoscope comprising:
- a catheter comprising a distal end and a plasma carrying lumen fluidly connected to said first lumen of said first flexible tube, for transporting a plasma generated by the plasma generating system to said catheter distal end;
- said electrically conductive means extending at least partially inside said first lumen and at least partially inside the plasma carrying lumen.

Preferably, said first lumen of said first flexible tube is fluidly connected to said plasma carrying lumen of said catheter. Said catheter being inserted inside said working channel of the endoscope such that there is a continuous fluidic connection along said first lumen of said first flexible tube and said plasma carrying lumen of the catheter for transporting a plasma generated by the plasma generating system to said catheter distal end. Preferably, said first lumen of said first tube and said plasma carrying lumen of said catheter are fluidly connected such that they are able to transport a plasma from said cold plasma chamber to said distal end of said catheter or of said endoscope. In another embodiment, said flexible tube enters the endoscope through said working channel down to said endoscope distal end. For example said catheter is said first flexible tube and is directly connected to said dielectric chamber and enters the endoscope through the working channel down to the endoscope distal end.

Preferably, the catheter of the invention is a tube that is inserted in the endoscope. Preferably, said catheter being inside said endoscope. Preferably, said catheter being inserted inside a working channel of said endoscope. According to another embodiment, the first flexible tube and the catheter are one single tube. The proximal end of such single tube is fluidly connected to the plasma chamber such that a plasma generated by the plasma generating system can be transported to said single tube distal end.

Preferably, said electrically conductive means extend along said first flexible tube and/or in said catheter inside said plasma carrying lumen.

Preferably, said first flexible tube being fluidly connected to said plasma carrying lumen of said catheter and said conductive wire extends along said first flexible tube and in said plasma carrying lumen of said catheter. In another embodiment, said conductive wire extends at least partially inside said single tube.

Preferably, said electrically conductive means extending at least partially inside said first lumen and at least partially inside the plasma carrying lumen are placed partially in said dielectric chamber, in said first lumen and at least partially in said plasma carrying lumen.

Preferably, said electrically conductive means extending at least partially inside said first lumen and at least partially inside the plasma carrying lumen are electrically insulated from said first electrode.

Preferably, said electrically conductive means extend essentially all along said first lumen and said plasma carrying lumen.

Preferably, said electrically conductive means extending at least partially inside said first lumen and at least partially inside the plasma carrying lumen are an electrically conductive wire.

Preferably, said electrically conductive wire extending at least partially inside said first lumen and at least partially inside the plasma carrying lumen is mechanically floating within said first lumen and within said plasma carrying lumen or said electrically conductive wire is mechanically coupled inside said first lumen and inside said plasma carrying lumen to a surface of said first lumen and of said plasma carrying lumen. Preferably, said electrically conductive wire is not electrically insulated within the first lumen and within the plasma carrying lumen.

In another embodiment, said catheter further comprises:
- a second lumen adjacent to said plasma carrying lumen for carrying a gas to said catheter distal end, said gas comprising one or more of the following gases: O₂, He, CO₂, H₂O vapour. This gas list being non-exhaustive and being cited as an example. This gas can be provided as a mixture of at least two gases, for example two of said gases.

The advantage of delivering a specific gas close to the plasma transported inside the plasma carrying lumen is to allow the formation of other reactive species than the ones constituting the original plasma. These other reactive species are generated by the reaction of the gas delivered by the second lumen with the plasma. These formed reactive species are preferably better suited than the reactive species of the plasma gas itself to treat specific cells. For example, the reaction of O₂ gas with the He plasma is particularly interesting for the treatment of gastrointestinal tract in order to smoothly resurface its mucosa.

Preferably said second lumen is configured to carry a gas to said plasma exiting said plasma carrying lumen at the level of the endoscope distal end.

Preferably,
- said catheter distal end or said endoscope distal end further comprises deployable confinement means for confining a plasma at least partially within said confinement means,
- said catheter further comprises a third lumen for carrying deployment means, said deployment means being:
   a fluid for deploying said confinement means by means of inflating it with said deployment fluid,
      or,
   a cable for deploying said confinement means by means of a displacement of said cable relative to said deployment means.

Preferably, said third lumen is adjacent to said plasma carrying lumen. Preferably, said third lumen is comprised within said multi-lumen catheter. Preferably, said deployable confinement means are deployable longitudinally, that is to say that the confinement means are deployable distally from the catheter distal end or endoscope distal end.

Confinement means of the plasma are particularly interesting for the resurfacing of the gastrointestinal tract in order to confine longitudinally the transported plasma and/or the reactive species generated by the plasma in order to treat efficiently a specific zone of the gastrointestinal tract mucosa. The endoscope with the confinement means of the invention allows to ensure the homogeneity of the treated zones through the diffusion of the ionized gas in the confinement structure. The method of the invention allows smooth resurfacing of the mucosa of the gastrointestinal tract with a mechanism of the plasma/reactive species on cells, triggering a cell-induced death (apoptosis) without inflammation, hence reducing post-operative complications. The procedure for smoothly resurfacing the gastrointestinal tract is predicted to be faster than existing technique, typically below one hour and more preferably below 30 minutes, given the short amount of time needed to dispense a large amount of plasma.

Preferably said deployment fluid is a gas. For example said deployment fluid is N₂.

Preferably, said confinement means comprises a first confinement means portion and a second confinement means portion,
said first confinement means portion being proximal to said endoscope and said second confinement means portion being distal to said endoscope,
said first and second confinement means portions are configured such that there exists openings between them when they are deployed by said deployment means.

For example the first and second confinement means portions are deployed in a gastrointestinal tract such that it allows to confine the plasma transported by the plasma carrying lumen in between them. The first and second confinement means portions are preferably deployed in contact with the gastrointestinal tact in order to confine as much as possible a gas or plasma injected between them. The confinement is created by the pressure exerted by the first and second confinement means portions on the gastrointestinal tract. This configuration of confinement means is particularly interesting because it possibly allows to build up a pressure between the first and second confinement means portions higher than the atmospheric pressure, allowing to expand the gastrointestinal tract locally such that gastrointestinal folds are unfolded such that they can be treated efficiently and homogeneously compared to other gastrointestinal portions. The pressure is built up thanks to the gas/plasma flows coming from the first and second lumen that are fluidly connected to the in between first and second confinement means portions.

Preferably, said catheter distal end or said endoscope distal end further comprises dispensing means fluidly connected to said plasma carrying lumen. Preferably, said dispensing means are radial dispensing means.

The advantage of dispensing means fluidly connected to the transporting means is to allow to treat large surfaces in an efficient way with the transported plasma.

Preferably, said dispensing means are configured for distributing radially a plasma transported by said plasma carrying lumen.

The advantage of dispensing means fluidly connected to the plasma carrying lumen is to allow to treat large surfaces in an efficient way with the transported plasma.

Preferably said plasma is transported by said first flexible tube and/or said catheter along a direction essentially tangent to said tube or catheter. Radially means that the plasma is dispensed radially around said direction tangent to said tube or catheter. Preferably radially means that the plasma is dispensed radially and perpendicularly around said direction tangent to said tube or catheter.

The advantage of the conductive means (for example a copper wire) is essentially to transport (it also allows to have an intense plasma and therefore enough to treat a living surface by plasma) but the advantage of the plasma endoscopic system for the treatment of the duodenum is due to the application system (confinement means and dispensing means) which makes it possible to treat large portions of tissue/mucosa. The combination of conductive means, confinement means and dispensing means is particularly synergetic and effective for cold plasma treatment of the gastrointestinal tract and more particularly of the duodenum tract. Thus this allows a time effective, homogeneous and qualitative treatment as well as ease of use for the practician (the practician only having to send the gas/plasma rather than having to steer manually a plasma carrying means all over a hollow body to be treated).

Preferably, said dispensing means comprises at least two holes, said at least two holes being configured for distributing said plasma transported by said plasma carrying lumen in a direction being essentially radial to a direction tangent of said plasma carrying lumen at the catheter distal end. Said holes being apertures in said dispensing means.

Preferably said plasma is transported by said flexible tubing along a direction tangent to said tubing. Radially means that the plasma is dispensed radially around said direction tangent to said tubing. Preferably said direction tangent to said plasma carrying lumen is taken at the catheter end.

Flexible tubes comprise one or more lumens. Lumens are the inner spaces in tubes that transport liquids, gases or surgical devices during an imaging or medical procedure. There exists single lumen tubes or multiple lumens, with multiple configurations and numbers. A catheter with a single hole through the centre of it, is referred to as a single lumen catheter or single lumen flexible tube. Multi-lumen catheters or multi-lumen flexible tubes have two or more lumens of varying sizes and shapes. The shape of a lumen being essentially defined by its cross-section. There also exists multi-lumen lined flexible tubes having a second material incorporated into one or more linings of the lumen, for example, the lining being the outer layer of the tube.

Preferably said endoscope comprises a working channel in which said catheter is positioned. For example said catheter is a multi-lumen catheter.

According to a third aspect, the invention relates to a method for cold plasma generation and transport, said method comprising the following steps:
a) providing a plasma generator comprising:
   ∘ a gas source,
   ∘ an electrical source,
   ∘ a plasma generating chamber comprising:
      ▪ a dielectric chamber fluidly connected to said gas source,
      ▪ a first electrode surrounding at least partially said dielectric chamber and electrically connected to said electrical source;
b) fluidly connecting a first flexible tube having a first lumen to said dielectric chamber; as
c) positioning an electrically conductive means partially in said dielectric chamber and at least partially in said first lumen, said electrically conductive means being electrically not connected from said electrical source;
d) generating a gas flow from said gas source through said dielectric chamber and said first lumen and applying an AC or pulsed electric potential to said first electrode for generating a plasma in said dielectric chamber, said generated plasma being transported through said first lumen by said gas flow.

Preferably, said plasma generating chamber comprises a grounded second electrode and said voltage source being electrically connected to the ground. For example, for a voltage source having a neutral and a phase, the neutral of a voltage source is grounded.

Preferably, said flexible tubing is a PTFE tubing having a diameter comprises between 1 mm and 5 mm, more preferably with a diameter of 2 mm.

Preferably said conductive wire is metallic, more preferably said conductive wire is a copper wire. Said metallic or copper wire preferably has a diameter comprised between 0.1 mm and 0.5 mm, more preferably it has a diameter of 0.2 mm.

The advantages described for the device for plasma treatment according to the first aspect and for the Plasma endoscopic system according to the second aspect of the invention also apply to the method for cold plasma generation and transport according to the third aspect, *mutatis mutandis.* The different variants for the device for plasma treatment according to the first aspect and for the Plasma endoscopic system according to the second aspect of the invention as described in the preceding text also apply to the method for cold plasma generation and transport according to the third aspect of the invention, *mutatis mutandis.*

According to a fourth aspect, the invention relates to a plasma endoscopic system comprising:
- an endoscope, said endoscope having a distal end,
- a catheter comprising a distal end and a plasma carrying lumen for delivering a plasma generated by a plasma generating system;
said catheter distal end or said endoscope distal end further comprises:
- confinement means for confining a plasma at least partially within said confinement means;
- dispensing means fluidly connected to said plasma carrying lumen.

The advantages described for the device for plasma treatment according to the first aspect, for the Plasma endoscopic system according to the second aspect of the invention, and for the method for cold plasma generation and transport according to the third aspect also apply to the plasma endoscopic system according to the fourth aspect, *mutatis mutandis.* The different variants for the device for plasma treatment according to the first aspect, for the Plasma endoscopic system according to the second aspect of the invention, and for the method for cold plasma generation and transport according to the third aspect also apply to the plasma endoscopic system according to the fourth aspect, *mutatis mutandis.* Advantages and variants described with the conductive means may apply without the conductive means, when the conductive means does provide the advantage, *mutatis mutandis.* Advantages and variants of the confinement and dispensing means described in combination with the conductive means also apply without the conductive means

An advantage of this fourth aspect of the invention over WO 2009/050240 A1 or other state of the art document is to allow a diffuse and radial plasma plume/plasma generated radical species delivery. In fact WO 2009/050240 A1 only allows a punctual/restricted and axial plasma plume delivery.

Preferably, said plasma generating system is the device for cold plasma treatment according to the first aspect of the invention or any other cold plasma generating device.

Preferably, said confinement means being deployable and said endoscope, more preferably said catheter, further comprises :
- a third lumen for carrying deployment means, said deployment means being:
   o a deployment fluid for deploying said confinement means by means of inflating it with said deployment fluid,
      or,
   ∘ a cable for deploying said confinement means by means of a displacement of said cable relative to said deployment means.

Preferably said confinement means comprises:
- a first confinement means portion and a second confinement means portion, said first confinement means portion being proximal to said endoscope and said second confinement means portion being distal to said endoscope,
   said first and second confinement means portions are configured such that there exists openings between them when they are deployed by said deployment means.

Preferably, said catheter distal tip or said endoscope distal tip further comprises dispensing means fluidly connected to said plasma carrying lumen.

Preferably, said dispensing means are configured for distributing radially a plasma transported by said plasma carrying lumen.

Preferably, said dispensing means comprises at least two holes, said at least two holes being configured for distributing said plasma transported by said plasma carrying lumen in a direction being essentially radial to a direction tangent of said plasma carrying lumen at the catheter distal end.

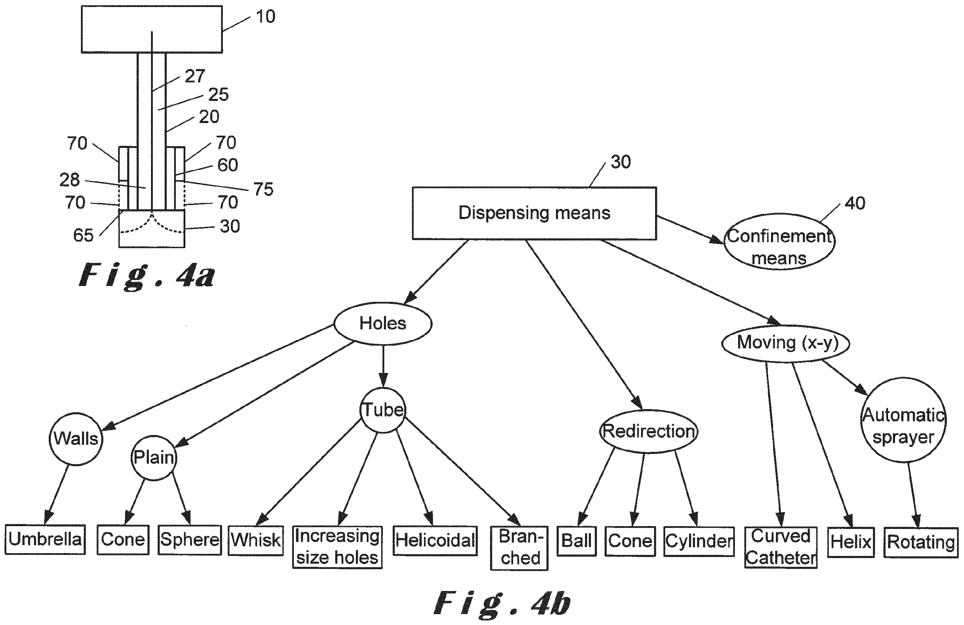

Preferably, said dispensing means comprises any one of the embodiments or variants of Fig. 4a to Fig. 9.

Preferably, said dispensing means comprises a plurality of bendable tubes fluidly connected by their proximal ends to the plasma carrying lumen and mechanically coupled at their distal ends to a foldable foil, said foldable foil having a distal part mechanically coupled to said cable for deploying said deployment means, said bendable tubes being deployed by pulling the distal part toward the proximal ends of the bendable tubes by pulling the cable, the deployed dispersing means being able to dispense radially the generated and transported plasma.

Preferably, said dispensing means comprises a plurality of bendable tubes fluidly connected by their proximal ends to the plasma carrying lumen and mechanically coupled at their distal ends, said plurality of bendable tubes comprising a plurality of holes on their outer sides such that plasma is dispensed radially around said bendable tubes.

Preferably, said confinement means comprises a cage formed by a plurality of bendable rods having distal and proximal ends, their proximal ends being mechanically coupled to the catheter or endoscope ends and their distal ends being mechanically coupled to each other's, said confinement means further comprise a first confinement portion formed by a first deployable foil mechanically coupled to a proximal portion of said plurality of bendable rods and a second deployable foil mechanically coupled to a distal portion of said plurality of bendable rods, said first and second deployable foils of said confinement means are configured such that there exists openings between them when they are deployed.

The advantage of the plasma endoscopic system for the treatment of the gastrointestinal tract is due to the application system (confinement means and dispensing means) which makes it possible to treat large portions of tissue/mucosa. The combination of confinement means and dispensing means is particularly synergetic and effective for cold plasma treatment of the gastrointestinal tract and more particularly of the duodenum tract. Thus this allows a time effective, homogeneous and qualitative treatment as well as ease of use for the practician (the practician only having to send the gas/plasma rather than having to steer manually the plasma carrying means all over a hollow body to be treated).

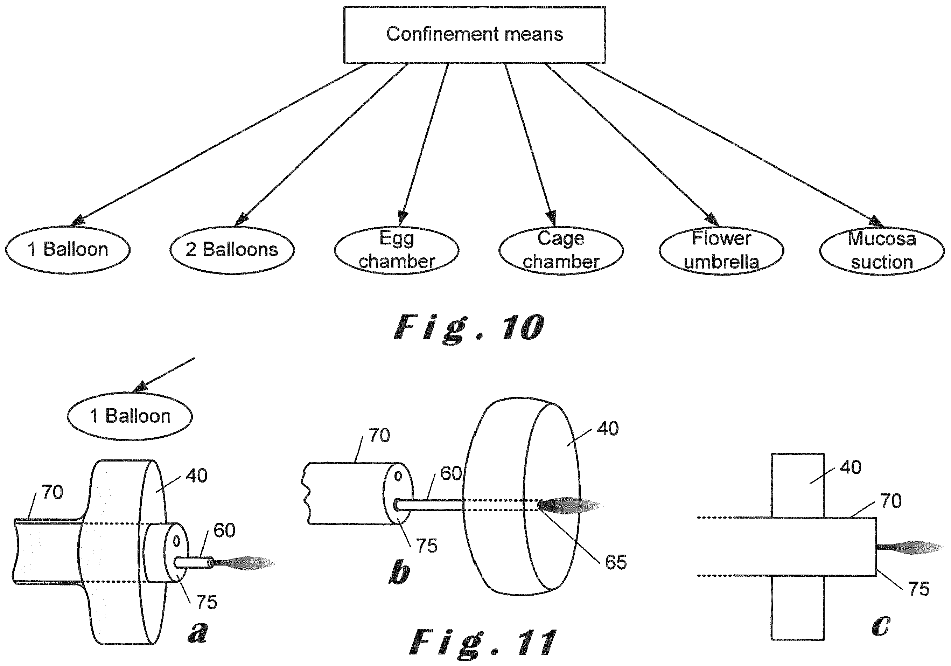

Preferably, said confinement means comprises any one of the embodiments or variants of Fig. 10 to Fig. 16.

Preferably, said plasma generating system comprises:
∘ a gas source,
∘ an electrical source,
∘ a plasma chamber comprising:
   ▪ a dielectric chamber fluidly connected to said gas source,
   ▪ a first electrode surrounding at least partially said dielectric chamber and electrically connected to said electrical source;
said plasma chamber being fluidly connected to said plasma carrying lumen.

Preferably, said plasma endoscopic system comprises an electrically conductive means extending at least partially inside said plasma carrying lumen.

Preferably, said electrically conductive means are placed partially in said dielectric chamber and at least partially in said plasma carrying lumen.

Preferably, said electrically conductive means are electrically insulated from said first electrode.

Preferably, said electrically conductive means extends essentially all along said plasma carrying lumen.

Preferably, said electrically conductive means are an electrically conductive wire. Preferably, said electrically conductive wire is not electrically insulated within the first lumen. Said electrically conductive wire is not electrically insulated from the gas and/or plasma inside said first lumen. Said electrically conductive wire is not insulated from said first flexible tube walls, for example first flexible tube walls being in PTFE.

Preferably, said electrically conductive wire is mechanically floating within said plasma carrying lumen or said electrically conductive wire is mechanically coupled inside said first lumen to an inner surface of said first flexible tube.

The addition of said conductive wire to said endoscope according to the fourth aspect of the invention shows a major advantage because it allows to achieve a combined technical effect with the application system.

### Brief description of the figures

These aspects of the invention as well as others will be explained in the detailed description of specified embodiments of the invention, with reference to the drawings in the figures, in which:
- Fig. 1 shows an exemplary embodiment of the device for plasma treatment according to the first aspect of the invention;
- Fig. 2 shows an exemplary embodiment of the plasma endoscopic system according to the second aspect of the invention;
- Fig. 3a-3c shows exemplary embodiments of the first flexible tube or of the catheter according to the invention;
- Fig. 4a, 4b show exemplary embodiments of the device according to the second aspect of the invention;
- Fig. 5a-5f show exemplary embodiments of the dispensing means of the device according to the second aspect of the invention;
- Fig. 6a, 6b show exemplary embodiments of the dispensing means of the device according to the second aspect of the invention;
- Fig. 7a-7e show exemplary embodiments of the dispensing means of the device according to the second aspect of the invention;
- Fig. 8a-8c show exemplary embodiments of the dispensing means of the device according to the second aspect of the invention;
- Fig. 9a-9c show exemplary embodiments of the dispensing means of the device according to the second aspect of the invention;
- Fig. 10 shows exemplary embodiments of the confinement means of the device according to the second aspect of the invention;
- Fig. 11a-11c show exemplary embodiments of the confinement means of the device according to the second aspect of the invention;
- Fig. 12a-12d show exemplary embodiments of the confinement means of the device according to the second aspect of the invention;
- Fig. 13, 14 show exemplary embodiments of the confinement means of the device according to the second aspect of the invention;
- Fig. 15, 15b, 16 show exemplary embodiments of the confinement means of the device according to the second aspect of the invention.

The drawings in the figures are not to scale. Generally, similar elements are designated by similar reference signs in the figures. The presence of reference numbers in the drawings is not to be considered limiting, even when such numbers are also included in the claims.

### Detailed description of possible embodiments of the invention

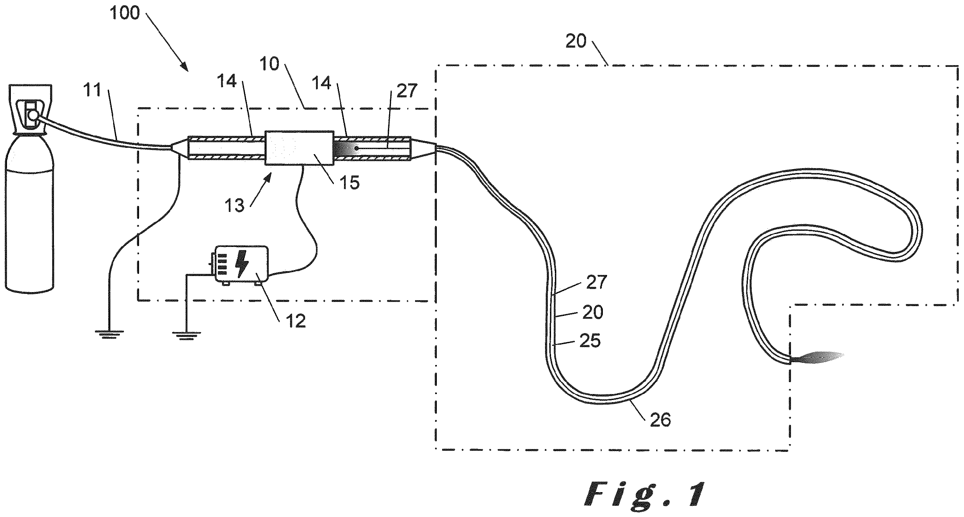

Figure 1 shows an exemplary embodiment of the device 100 for plasma endoscopy according to the invention. The device 100 comprises a plasma generating system 10 that is connected to a gas supply connected to a gas source 11. The gas flow of the gas source 11 is controlled to deliver a gas flow of about 0.5 to 5 L/min. The plasma generating system 10 comprises a dielectric chamber 14 into which the gas from the gas source 11 flows. For example the dielectric chamber 14 is a quartz cylinder closed at its two ends by two fluidic connections to the gas source 11 and to the first flexible tube 20. The dielectric chamber 14 is at least partially surrounded by a first electrode 15. For example said first electrode 15 is a conductive tape or pieces of conductive tape. For example the first electrode 15 is formed around the dielectric chamber 14. The first electrode 15 is electrically connected to an electrical source 12 and preferably to a voltage source. The first electrode 15 is connected to a voltage source such that the voltage source 12 is configured to vary the electric potential of said first electrode 12. For example the voltage source is an AC voltage source with a frequency comprised between 1 kHz and 1 MHz. In another example, the voltage source 12 is a pulsed voltage source that delivers voltage pulses having a voltage amplitude higher than 1 kV, more preferably higher than 3 kV and even more preferably higher than 5 kV, and with a pulse width comprised between 1 ns to 1 µs in the kHz range. In Fig. 1, the electrical source or voltage source is grounded as well as a second electrode positioned at least partially in contact with the dielectric chamber 14. When a high potential is applied to the first electrode, a dielectric barrier discharge occurs and the gas in the dielectric chamber is ionised into a cold plasma. Said plasma is an atmospheric plasma since it is formed at a nearly atmospheric pressure. With the gas flow generated by the gas source 11, the plasma flows toward the first flexible tube 20 into the first lumen 25 where it is transported until a first flexible tube end.

In Fig. 1, an electrically conductive means 27 is placed in said first lumen 25. Preferably, the electrically conductive means 27 is placed partially inside the dielectric chamber 14 but not in a portion of the dielectric chamber 14 surrounded by the first electrode 15. The electrically conductive means 27 goes inside the first lumen 25, in contact with the plasma that flows inside the first lumen 25. The electrically conductive means 27 are placed until approximatively the end of the first lumen 25. For example the electrically conductive means are positioned so that they end between 2 cm before the first lumen 25 end to 1 cm after, preferably between 1 cm before to 0.5 cm after and even more preferably between 0.5 cm before and 0 cm after. In Fig. 1, the device 100 is shown when functioning, the generated plasma inside the dielectric chamber is represented by the grey shades between the first electrode 15 and the proximal end of the electrically conductive means 27 and at the exit of the first flexible tube 20 by the plume in a grey shade. An analysis of the emission intensity of the plasma during its generation shows that the emission intensity is much lower all along the electrically conductive means 27, in this case a copper wire having a diameter of 0.2 mm. Then when the plasma exits the first lumen 25, with the copper wire stopping between 1 cm and 0 cm, preferably 5 mm before the end of the first lumen 25, the plasma turns ON, with an intensity similar to the intensity observed in the dielectric chamber 14, between the first electrode and the proximal end of the copper wire. A plurality of positioning means could be used to mechanically couple the electrically conductive means inside the first lumen 25 in order that it has a fixed proximal end and a fixed distal end regarding the dielectric chamber and the lumen end respectively.

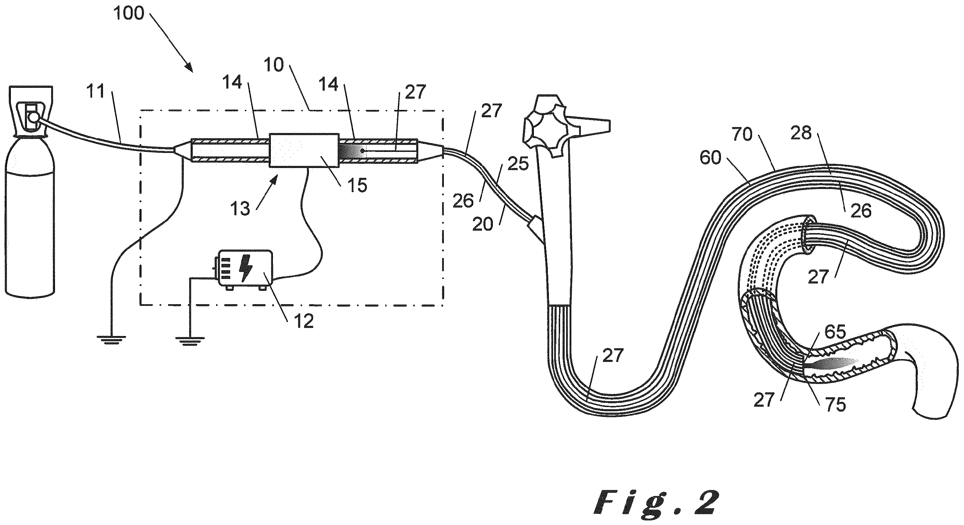

Figure 2 shows a plasma endoscopy system 200 comprising the device 100 shown in Fig. 1. The plasma endoscopy system 200 comprises an endoscope and the device 100 according to Fig. 1. The endoscope shows a control section for controlling the endoscope functions and guidance. The endoscope also shows an instrument channel or working channel. The device 100 is connected at this position of the endoscope of the flexible tube 20 is inserted as a catheter inside said working channel of the endoscope in order to deliver a plasma at the endoscope distal end. The endoscope insertion tube 70 or endoscope 70 that can be inserted into a hollow body comprises an outer envelope defining the outer contour of the endoscope 70. It further shows a working channel used to carry the plasma through the plasma carrying lumen 28, the plasma carrying lumen 28 being formed into a catheter 60. The catheter is a flexible tube, i.e. a single-lumen catheter or a multi-lumen catheter.

Figure 2 also shows another embodiment comprising a single tube fluidly connected to the plasma chamber 13 which allows to transport the generated plasma to the endoscope distal end 65. This single tube comprises the first tube 20 and the first lumen of the catheter 28. This single tube is preferably made in one single piece of tube, such that the first lumen 25 and the plasma carrying lumen 28 are within a single tube. As illustrated on Figure 2, the conductive means 27 extends at least partially inside the single tube lumen.

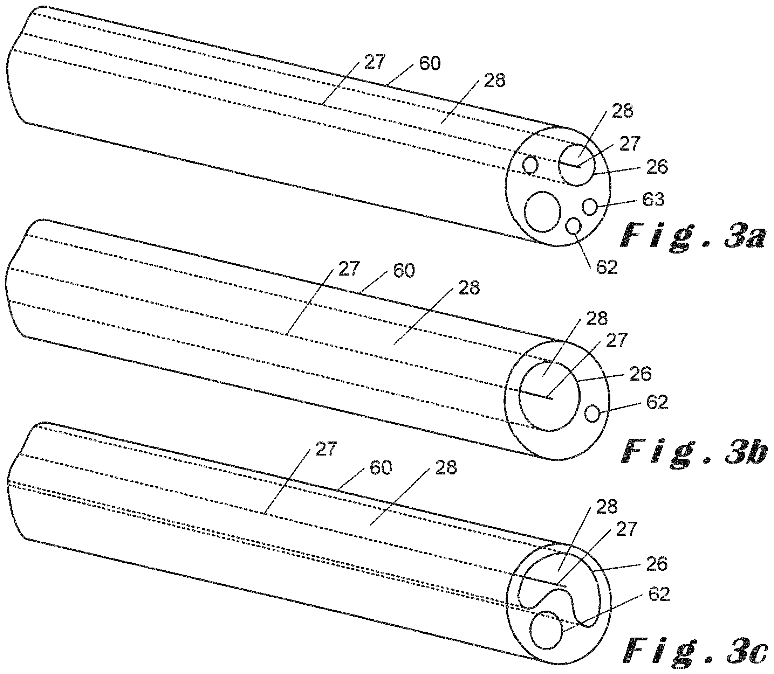

Figure 3a, 3b, and 3c show three examples of multi-lumen catheters 60 or flexible tube 20 that can be utilized within the scope of the invention. A plasma carrying lumen 28 is represented delimited by inside surface 26. An electrically conductive means 27 is also represented in dashed lines when it is inside the catheter 60 or tube 20 and with a solid line when it is directly observable. On Fig. 3a, a second lumen 62 is represented for carrying a gas, a third lumen 63 is also represented for carrying deployment means, for example a deployment fluid or a cable. Other lumens shown can be kept available for improvement of the present invention. Fig. 3b and 3c show a first 28 and a second 62 lumen having different shapes, depending on the ratio of plasma and gas to be delivered to the dispensing means 30. Fig. 3b and 3c do not show a third lumen 63 for carrying deployment means because said deployment means can also be carried through a third lumen 63 located inside another catheter or through an over-tube fluidic connection. An over tube fluidic connection is shown on Fig. 11a, 12a, and 16 where there is a gap between the endoscope 70 and an over tube wall.

Figure 4a shows a schematic embodiment of the second aspect of the invention. The first flexible tube 20 or the endoscope 60 having a fist lumen 25 carries the plasma generated by the plasma generating system 10 to the dispensing means 30. As it is shown on Fig. 4a, the distal end 65 of the catheter 60 either ends at the same point than the distal end of the endoscope 75 as represented by the dotted lines of the endoscope 70 or the distal end 65 of the catheter 60 ends further to the distal end 75 of the endoscope 70. The dispensing means 30 are mechanically and fluidly connected to the distal end of the catheter 60. The electrically conductive means 27 extends inside said dispensing means in order to transport the plasma and to turn on the plasma again just before exiting said dispensing means 30. Preferably the electrically conductive means 27 is divided into many wire in order to radially distribute the plasma. For example the dispensing means 30 comprises a plurality of openings, therefore a plurality of electrically conductive means 27 extends in the direction of each of said openings.

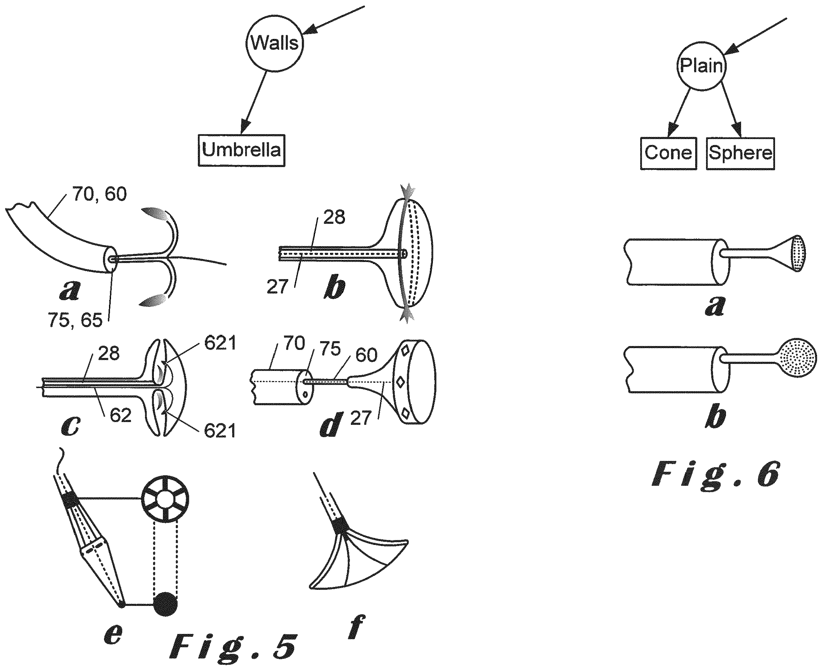

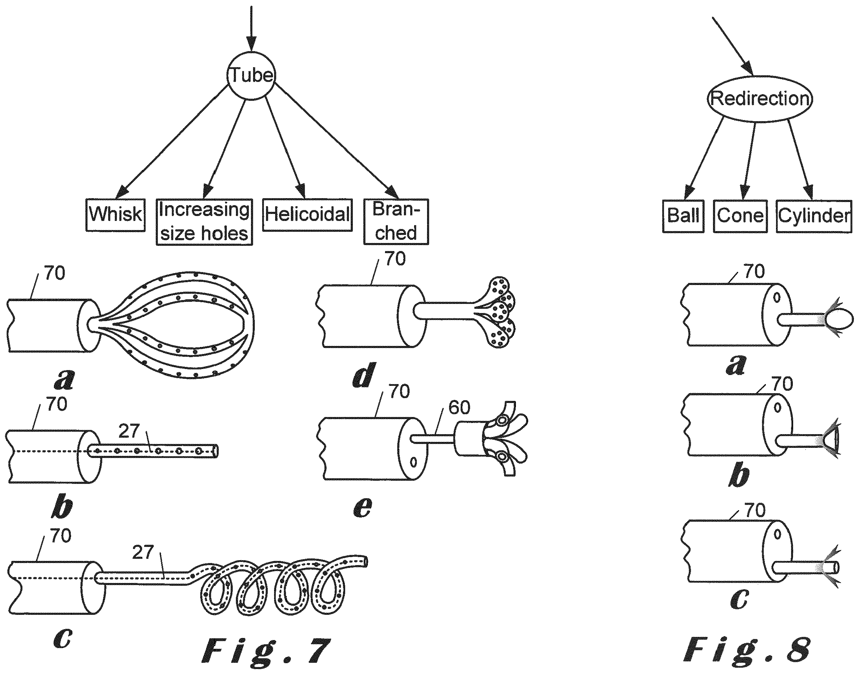

Figure 4b shows a chart showing many dispensing means 30 alternatives in a hierarchical way. The dispensing means 30 described in this chart are sought to allow an homogenous and radial distribution of the plasma into a hollow body. This chart can be read as follows: a dispensing means 30 can have multiple holes, one hole with moving (x-y) means, one or multiple holes with redirecting means or either only a longitudinal confinement means 40 also able to dispense radially the plasma.. In the case of multiple holes, these holes can be located:
- around the walls of the dispensing means 30 (see Fig. 5);
- in a plan or in a curved surface (see Fig. 6);
- in a tube, the tube having different shapes and or tube subdivisions (see Fig. 7).

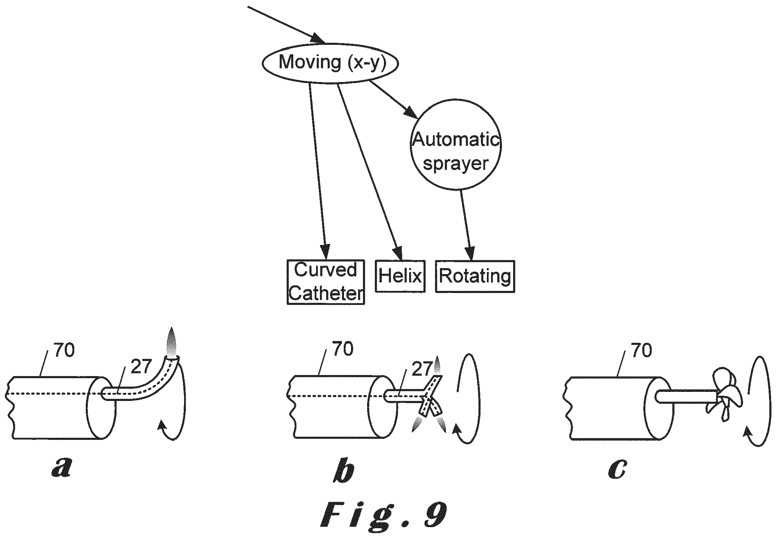

In the case of redirecting means (see Fig. 8) the plasma is redirected by a cap. In the case of moving (x-y) means, the plasma nozzle is or are rotated such as to dispense the plasma radially around a rotation axis (see Fig. 9).

The plasma can also simply be dispensed homogenously in a hollow body thanks to confinement means 40 that allow plasma or reactive species to reach each point of the hollow body thanks to the plasma flow from said plasma carrying lumen 28.

Figure 5 a shows a dispensing means 30 with at least two tubes fluidly connected to the plasma carrying lumen 28, these tubes are preferably self-expandable or having a pre-formed shape such that the plasma can be dispensed around the catheter 60 after self-expansion or after having retrieved said preformed shape. The self-expandability or ability to retrieve said preformed shape allows the tubes to deploy by itself. Fig. 5b shows a dispensing means 30 comprising a head into which a groove is formed, said groove being fluidly connected to said plasma carrying lumen 28. Said groove allowing to dispense a plasma coming from the plasma carrying lumen 28 all around the endoscope 70. The electrically conductive means 27 is shown within the plasma carrying lumen 28, which can be prolonged within said groove into multiple electrically conductive means, for example coated on the groove surface. Fig. 5c shows a head similar to the one of Fig 5b, Fig. 5b is a perspective view while Fig. 5c being a cross-sectional view. Head of Fig. 5c comprising gas nozzles 621 within said groove of said head in order to provide gas from the second lumen 62. The gas nozzles 621 being fluidly connected to the second lumen 62. The plasma carrying lumen 28 is shown as well as the second lumen 62 adjacent to each other's within the catheter 60. The plasma shown by the grey shading can be seen to disappear after the gas from the second lumen 62 is injected. This is because the plasma has reacted with the gas from the second lumen in order to form reactive species. Preferably these reactive species are free radicals. Free radicals are preferably neutral. Therefore no emission spectrum can be observed from said reactive species. Fig. 5d shows a head with a plurality of diamonds openings around the radial surface of the head. Any other opening shapes can be used. Diamonds are here simply shown as an example, openings can be for examples, triangles, slits, squares, rectangles, polygons, holes,... For example, there are between 4 to 16 diamonds holes all-around said radial surface. Preferably this dispensing mean 30 extends further to said endoscope and is mechanically coupled to the catheter 60. The electrically conductive means 27 extends within said head and preferably a plurality of electrically conductive means 27 extend to the opening of the radial surface of the head. Dispensing means 30 with a head being for example deployable or inflatable by means of a deployment fluid.

Fig. 5e and Fig. 5f show a same folded and unfolded dispensing means 30 respectively. This shown dispensing means 30 is similar to an inverted umbrella. The dispensing means comprises a plurality of rigid flexible tube like umbrella ribs fluidly connected to the plasma carrying lumen 28. The dispensing means 30 of Fig. 5e is deployed by pulling the distal part represented by a black disk on the right hand-side of the Fig. 5e to the centre of the open ring shown on the right-hand side of Fig. 5e. The distal part of the dispensing means 30 is pulled to the proximal deployment mean (open ring) by a cable carried in said third lumen 63. On Fig. 5f, the plasma can then be dispensed radially to said endoscope, with a rigid umbrella structure. Such a dispensing can comprise between 4 to 12 umbrella like ribs for deploying and prolong the plasma carrying lumen 28.

Fig. 6a shows a dispensing means 30 where a watering can rose type of head is used to homogenously dispense the plasma. The plurality of holes is formed on a curved surface. A tangent of the curved surface in its centre being perpendicular to the main direction of the catheter 60. Dozens of holes can be formed on said curved surface. Fig. 6b, shows a dispensing means 30 having a shower head type of dispensing means 30. The holes being formed on a flat or curved surface. A tangent to the centre of the surface being parallel to the main direction of the catheter 60.

Fig. 7 show different variants of tubes with holes for dispensing the plasma. Fig. 7a shows a whisk, with each wire of the whisk being tubes fluidly connected to the plasma carrying lumen 28. Each whisk tube being perforated on its external surface in order to deliver the plasma radially. Preferably the whisk tubes are flexible such that it can easily be inserted into a hollow body. Such a dispensing means 30 comprises at least four tube whisks and for example 5, 6, 7, 8, 9, or 10. Fig. 7b shows a dispensing means 30 being a single tube prolonging the plasma carrying lumen 28 of the catheter 60. The tube being perforated with holes of diameter larger at the distal part of the tube than at its proximal part. The tube being blocked at its end. The increasing hole diameter when going toward the distal part allows to compensate pressure drop caused by the respective proximal holes. Fig. 7c is similar to Fig. 7b but the tube is coiled into a helicoidal shape. The tube is preferably blocked at its end. The holes are preferably formed on the external surface of the tube. Fig. 7d shows a branched like shape for dispensing the plasma in a hollow body. Fig. 7d dispensing means comprises branches elongating in the distal direction, each branch having a plurality of holes. Preferably an electrically conductive means 27 extends into each of said branches. Fig. 7e shows a similar dispensing means than in Fig. 7d but the branches are open ended with large holes, for example the diameter of the large holes is similar to the diameter of the plasma carrying lumen 28.

Fig. 8a, b, c show compact dispensing means 30 having a main opening fluidly connected to the plasma carrying lumen 28 in front of which a redirecting means is placed such that the flow of plasma exiting the opening hits the redirecting means and flows around it in order to be spread around it. Fig 8a redirecting means is a ball, oval, round, sphere or ellipsoid. Fig. 8b is a cone, the top of the cone being oriented to the plasma flow. Fig. 8c redirecting means is a cylinder, preferably the cylinder having a diameter equal to that of the main opening.

Fig. 9 shows three embodiments of dispensing means 30 with a rotating part in order to distribute a plasma radially in an homogeneous way. Fig. 9a shows a curved tube that is rotatably mounted on said endoscope and fluidly connected to the plasma carrying lumen 28. Rotating means are provided through a lumen of the catheter of through a channel of the endoscope and is controlled on the control section of the endoscope 70. For example, rotating means are activated by twisting the part of the catheter that remains out of the endoscope into the practitioner hand. Fig. 9b shows a multiple tubes having at least three tubes fluidly connected to the plasma carrying lumen 28 allowing a similar dispersion of the plasma than Fig. 9a but with a lower rotating speed. Fig. 9c shows a straight tube with a single opening with an helix or a propeller rotatably mounted distally to the opening in order to spray around the plasma.

Fig. 10 shows a chart showing the confinement means 40 configurations envisaged by the inventors. The confinement means are described in detail in Fig. 11 to Fig. 16.

Fig. 11a and 11b show a confinement means with one balloon. In Fig. 11a, the single balloon is positioned around the endoscope 70, allowing to have vision where the plasma is delivered. Fig. 11b shows a balloon positioned around said catheter 60, the catheter distal end 65 being distal to the balloon. In Fig. 11b, the balloon is over the catheter 60. The balloon of Fig. 11a and 11b being inflatable by connecting them fluidly to the third lumen 63 and inflating them with a deployment means such N₂, CO₂, or air. Fig. 11c shows a schematic representation of Fig. 11a. In another embodiment, said third lumen 63 is another catheter or a flexible tubing next to the endoscope 70 and is not within the catheter 60.

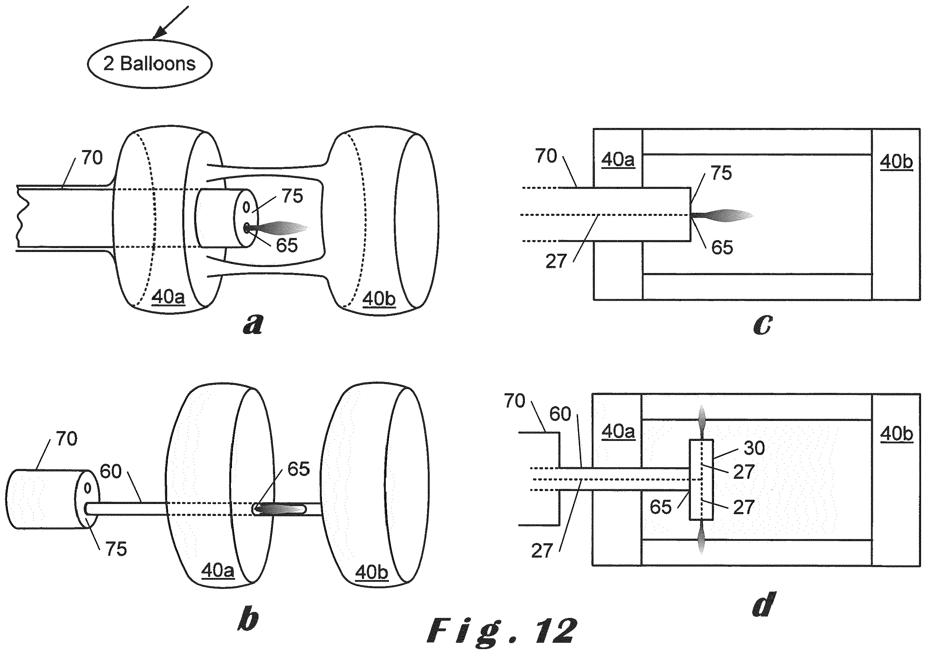

Fig. 12 shows two balloons for confining a volume defined by walls and said balloons, said walls to be treated with the plasma or with reactive species generated by reaction with the plasma. The first balloon is a first confinement means portion 40a and the second balloon is a second confinement means portion 40b. The first balloon 40a of Fig 12a and Fig 12b is the same than the balloon of Fig 11a and Fig. 11b respectively. The second balloon 40b is mechanically connected to the first balloon 40a in order to keep space between the balloons 40a, 40b. In Fig 12a the mechanical connection is at least two filaments in which air can pass from the first to the second balloon such that both balloon can be inflated from the same deployment means, for example deployment means from the third lumen 63 or from another catheter/flexible tube. In Fig. 12b, the mechanical connection is at the level of the catheter 60 such that the third lumen 63 of the catheter can inflate both balloons. For example, in Fig 12b, mechanical connections are at least two catheters such as in Fig. 12a. In Fig. 12b, the first balloon is over the endoscope 70. Fig. 12c shows a schematic view of Fig. 12a. Fig. 12d shows a schematic view of Fig. 12b, with in addition a dispensing means allowing a radial dispensing of the plasma.

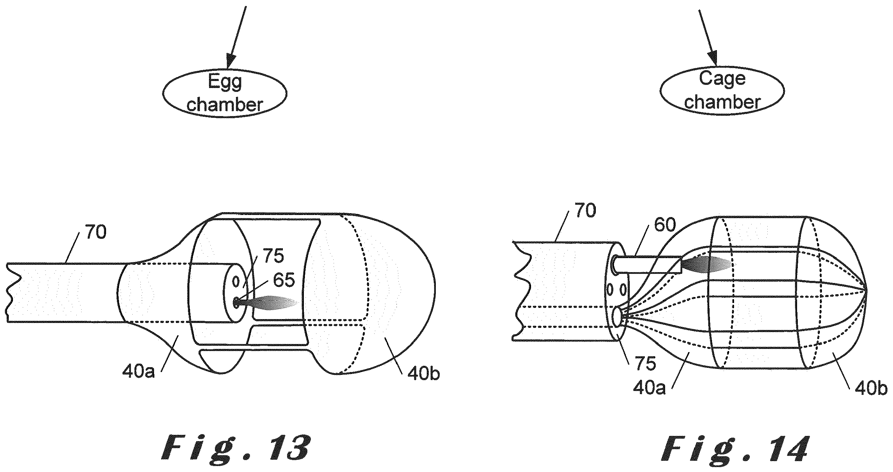

Fig. 13 shows a confinement means 40 with an egg shape having opening in order to be able to treat a confined surface. The confinement means 40 of Fig. 13 comprises a first confinement means portion 40a being proximal and a second confinement means portion 40b being distal. The two portions 40a, 40b being mechanically coupled by filaments or ribs having a self-expandable or having a pre-formed shape such that the plasma can be dispensed around the catheter 60 / endoscope 70 after self-expansion or after having retrieved said preformed shape. The self-expandability or ability to retrieve said preformed shape allows the two portions to deploy by itself. The endoscope distal end 75 and catheter distal end 65 being in between the two portions 40a and 40b such that vision is possible and plasma and gas delivery occur in the confined spaced. The two portions 40a, 40b being in a foldable material, the two portions 40a, 40b can be stretched by applying a higher pressure in between them, by means of the plasma flux or by means of the gas from the second lumen 62, or, by means of the auto/self-expandability of their constitutive material.

Fig. 14 shows a confinement means 40 with a cage chamber, the distal and proximal ends of the cage chamber being the a first confinement means portion 40a and a second confinement means portion 40b respectively. The cage comprises ribs made in a material being self-expandable or having a pre-formed shape, for example a material used for stents, *i.e.* self-expandable polymers such as polyesters. The distal 40a and proximal 40b ends being made in a foldable material such as a plastic foil or a coated mat, or a silicone (polysiloxanes). The catheter distal end 65 being positioned within said confined space between said distal 40a and proximal 40b ends. The ribs are preferably preformed and deployable. For example their deployment can be triggered by the deployment means from the third lumen 63, for example a cable or self-expandable (thanks to the elasticity of the material). This embodiment of Fig. 14 allows to treat a hollow surface at a desired position in an easy way and allows to displace within the hollow body the deployed confinement means 40 where the hollow body needs to be treated. Large surface area of hollow body to be treated can be reached in a relatively short time with the embodiment of Fig. 14. Thanks to this confinement means and dispersing means design, the dispersing means can be moved within the volume defined by the confinement means.

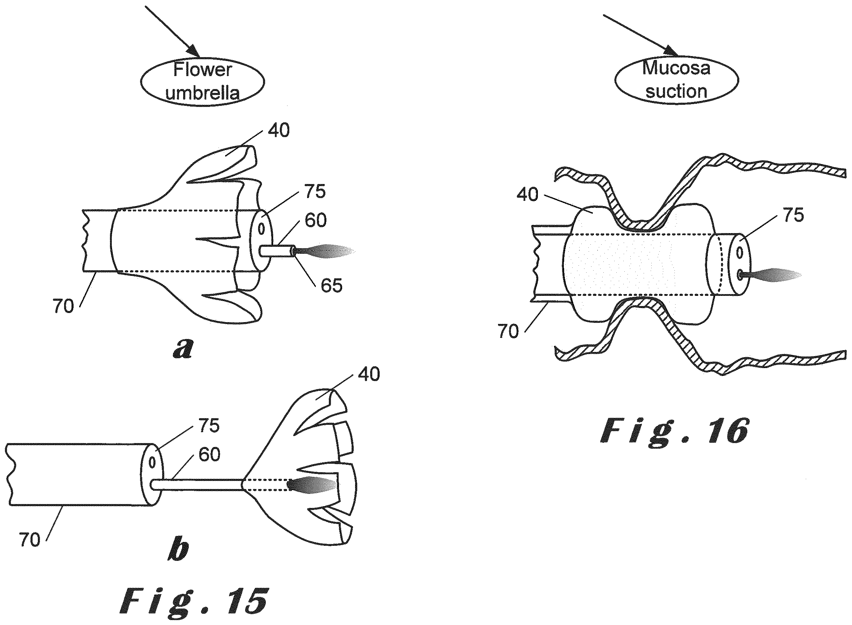

Fig. 15a shows a confinement means 40 being a flower type umbrella over the scope. This confinement means 40 can be deployed by means of inflating it or mechanically deploying it. Fig. 15b shows the same confinement means 40 than in Fig. 15a but instead of being over the endoscope 70, the confinement means 40 is deployed over the catheter 60.

Fig. 16 shows a confinement means that rely on suction of a portion of the mucosa in order to confine a portion of a hollow body. Suction can be carried out by means of holes located around the endoscope 70 and fluidly connected to the third lumen 63, said third lumen 63 being submitted to partial vacuum in order to create a mucosa suction. For this embodiment, said third lumen 63 being outside the endoscope as shown on Fig. 16. This embodiment can for example be combined with a distal balloon 40b as shown in Fig. 12a or Fig. 12b, the mucosa suction confinement means being a proximal confinement means 40a.

All the embodiments of dispensing means of Fig. 3 to 9 and all embodiment of confinement means of Fig. 10 to Fig. 16 can be combined.

The present invention has been described with reference to a specific embodiment, the purpose of which is purely illustrative, and they are not to be considered limiting in any way. In general, the present invention is not limited to the examples illustrated and/or described in the preceding text. Use of the verbs "comprise", "include", "consist of", or any other variation thereof, including the conjugated forms thereof, shall not be construed in any way to exclude the presence of elements other than those stated. Use of the indefinite article, "a" or "an", or the definite article "the" to introduce an element does not preclude the presence of a plurality of such elements. The reference numbers cited in the claims are not limiting of the scope thereof.

In summary, the invention may also be described as follows. Device 100 for plasma endoscopy comprising:
- a plasma generating system 10 comprising:
   ∘ a gas source 11,
   ∘ an electrical source 12,
   ∘ a plasma chamber 13 comprising:
      ▪ a dielectric chamber 14 fluidly connected to said gas source 11,
      ▪ a first electrode 15 surrounding at least partially said dielectric chamber 14 and electrically connected to said electrical energy source 12;
- a first flexible tube 20 having a first lumen 25 fluidly connected to said dielectric chamber 14;
   said device 100 further comprising:
- electrically conductive means 27 positioned at least partially inside said first lumen 25.

## Claims

1. Device (100) for cold plasma treatment comprising:
- a cold plasma generating system (10) comprising:
∘ a gas source (11),
∘ an electrical source (12),
∘ a cold plasma chamber (13) comprising:
▪ a dielectric chamber (14) fluidly connected to said gas source (11),
▪ a first electrode (15) surrounding at least partially said dielectric chamber (14) and electrically connected to said electrical source (12);
- a first tube (20) having a first lumen (25) fluidly connected to said dielectric chamber (14);
**characterized in that**
said device (100) further comprises:
- electrically conductive means (27) positioned at least partially inside said first lumen (25).

2. Device (100) according to the preceding claim **characterized in that** said electrically conductive means (27) are placed partially in said dielectric chamber (14) and at least partially in said first lumen (25).

3. Device (100) according to any one of the preceding claims **characterized in that** said electrically conductive means (27) are electrically insulated from said first electrode (15).

4. Device (100) according to any one of the preceding claims **characterized in that** said electrically conductive means (27) extend essentially all along said first lumen (25).

5. Device (100) according to any one of the preceding claims **characterized in that** said electrically conductive means (27) are an electrically conductive wire (27).

6. Device (100) according to the preceding claim **characterized in that** said electrically conductive wire (27) is mechanically floating within said first lumen (25) or said electrically conductive wire (27) is mechanically coupled inside said first lumen (25) to an inner surface (26) of said first flexible tube (20).

7. Cold plasma endoscopic system (200) comprising a device (100) according to any one of the preceding claims.

8. Cold plasma endoscopic system (200) according to previous claim **characterized in that** said plasma endoscopy system (200) comprises:
- an endoscope (70), said endoscope (70) having a distal end (75),
- a catheter (60) comprising a distal end (65) and a plasma carrying lumen (28) fluidly connected to said first lumen (25) of first flexible tube (20), for transporting a cold plasma generated by the cold plasma generating system (10) to said catheter distal end (65);
- said electrically conductive means (27) extending at least partially inside said first lumen (25) and at least partially inside the plasma carrying lumen (28).

9. Cold plasma endoscopic system (200) according to previous claim **characterized in that** said catheter (60) further comprises:
- a second lumen (62) adjacent to said cold plasma carrying lumen (28) for carrying a gas to said catheter distal end (65), said gas comprising one or more of the following gases: O₂, He, CO₂, H₂O vapour.

10. Cold plasma endoscopic system (200) according to any one of the two previous claims **characterized in that**:
- said catheter distal end (65) or said endoscope distal end (75) further comprises deployable confinement means (40) for confining a cold plasma at least partially within said confinement means (40),
- said catheter (60) further comprises a third lumen (63) for carrying deployment means, said deployment means being:
a fluid for deploying said confinement means (40) by means of inflating it with said deployment fluid,
or,
a cable for deploying said confinement means (40) by means of a displacement of said cable relative to said deployment means.

11. Cold plasma endoscopic system (200) according to previous claim **characterized in that**:
- said confinement means (40) comprises:
- a first confinement means portion (40a) and a second confinement means portion (40b),
said first confinement means portion (40a) being proximal to said endoscope (70) and said second confinement means portion (40b) being distal to said endoscope (70),
said first (40a) and second (40b) confinement means portions are configured such that there exists openings between them when they are deployed by said deployment means.

12. Cold plasma endoscopic system (200) according to any one of the four previous claims **characterized in that** said catheter distal end (65) or said endoscope distal end (75) further comprises dispensing means (30) fluidly connected to said cold plasma carrying lumen (28).

13. Cold plasma endoscopic system (200) according to previous claim **characterized in that** said dispensing means (30) are configured for distributing radially a cold plasma transported by said cold plasma carrying lumen (28).

14. Cold plasma endoscopic system (200) according to any one of the two previous claims **characterized in that** said dispensing means (30) comprises at least two holes, said at least two holes being configured for distributing said cold plasma transported by said cold plasma carrying lumen (28) in a direction being essentially radial to a direction tangent of said cold plasma carrying lumen (28) at the catheter distal end (65).

15. Method for cold plasma generation and transport, said method comprising the following steps:
a) providing a cold plasma generator (10) comprising:
∘ a gas source (11),
∘ an electrical source (12),
∘ a cold plasma generating chamber (13) comprising:
▪ a dielectric chamber (14) fluidly connected to said gas source (11),
▪ a first electrode (14) surrounding at least partially said dielectric chamber (14) and electrically connected to said electrical source (12);
b) fluidly connecting a first flexible tube (20) having a first lumen (25) to said dielectric chamber (14);
c) positioning an electrically conductive means (27) partially in said dielectric chamber (14) and at least partially in said first lumen (25), said electrically conductive means (27) being electrically not connected from said electrical source (12);
d) generating a gas flow from said gas source (11) through said dielectric chamber (14) and said first lumen (25) and applying an AC or pulsed electric potential to said first electrode (15) for generating a cold plasma in said dielectric chamber (14), said generated cold plasma being transported through said first lumen (25) by said gas flow.
